# EUROPEAN PATENT SPECIFICATION

(11) **EP 0 384 750 B1**
(45) Date of publication and mention of the grant of the patent: **02.11.1995**
(21) Application number: 90301898.4
(22) Date of filing: 22.02.1990
(51) Int. Cl.: C12N 15/53, C12N 9/02, C12N 1/21

(54) **Process of producing human 5-lipoxygenase**
Verfahren zur Herstellung von menschlicher 5-Lipoxygenase
Procédé pour la production de 5-lipoxygénase humaine

(30) Priority: 22.02.1989 JP 40206/89
(43) Date of publication of application: 29.08.1990
(73) Proprietor: Japan Tobacco Inc., Minato-Ku Tokyo 105 (JP)
(72) Inventor: Matsumoto, Takashi, Life Science Res. Lab., Yokohama-shi, Kanagawa 227 (JP); Noguchi, Masato, Life Science Res. Lab., Yokohama-shi, Kanagawa 227 (JP); Nakamura, Motonao, Life Science Res. Lab., Yokohama-shi, Kanagawa 227 (JP); Noma, Masana Life Science Res. Lab., Yokohama-shi Kanagawa 227 (JP)
(74) Representative: Paget, Hugh Charles Edward

(56) References cited:
- THE JOURNAL OF BIOLOGICAL CHEMISTRY, vol. 263, no. 21, 25th July 1988, pages 10135-10140, The American Society for Biochemistry and Molecular Biology, Inc.; C.A. ROUZER et al.: "Characterization of cloned human leukocyte 5-lipoxygenase expressed in mammalian cells"
- PROC. NATL. ACAD. SCI. USA, vol. 85, no. 1, pages 26-30, January 1988, Washington, DC, US; T. MATSUMOTO et al.: "Molecular cloning and amino acid sequence of human 5-lipoxygenase"
- FEBS LETTERS, vol. 249, no. 2, June 1989, pp. 267-270, Federation of European Biochemical Societies, Amsterdam, NL; M. NOGUCHI et al.: "Expression of human 5-lipoxygenase cDNA in Escherichia coli"
- PROC. NATL. ACAD. SCI. USA, vol. 86, April 1989, pages 2592-2596, Washington, DC, US; C.D. FUNK et al.: "Native and mutant 5-lipoxygenase expression in a baculovirus/insect cell system"

## Description

This invention relates to a process of producing human 5-lipoxygenase which is an enzyme which converts arachidonic acid to 5-hydroperoxyeicosatetraenoic acid and then converts 5-hydroperoxyeicosatetraenoic to leukotriene A₄.

5-Lipoxygenase is an enzyme which catalyzes the formation of 5-hydroperoxy-6,8,11,14-eicosatetraenoic acid (5-HPETE) from arachidonic acid and which further converts 5-HPETE to 5,6-oxido-7,9,11,14-eicosatetraenoic acid (leukotriene A₄). Leukotriene A₄ is then converted to peptide leukotriene or leukotriene B₄ by other enzymes. Thus, 5-lipoxygenase catalyzes the first two steps in the biosynthesis of the leukotrienes. 5-Lipoxygenase also participates in the formation of lipoxins. Although the bioactivity of leukotrienes and lipoxins has not yet been fully revealed, it is known that leukotrienes play important roles in the contraction of smooth muscle and in the induction of inflammation. Other important activities include enhancement of secretion in the bronchus mucosa, enhancement of secretion of insulin in the pancreas, enhancement of secretion of luteinizing hormone in the brain, stimulation of leucocytes, stimulation of suppressor T cells, stimulation of natural killer cells and enhancement of production of interferon. Thus, human 5-lipoxygenase may be used as a reagent for developing pharmaceuticals.

5-Lipoxygenase has been isolated from human leucocytes (Proceedings of the National Academy of Sciences, U.S.A., vol. 82, p. 6040 (1985)), porcine leucocytes (The Journal of Biological Chemistry, vol. 261, p. 7982 (1986)), from mast cells originated from mouse bone marrow (Proceedings of the National Academy of Sciences U.S.A., vol. 83, p.4175 (1986)) and from rat basophilic leukemia cells (Prostaglandins, vol. 29, p. 689 (1985)). Although the isolated 5-lipoxygenases are common in that they require calcium for the expression of their activities, other activation factors are different. Further, it is suggested that 5-lipoxygenase of different species is composed of different polypeptide because the molecular weight of the 5-lipoxygenase from human is 80,000, from porcine is 72,000, from mouse is 75,000, and from rat is 73,000. Therefore, up to now, there is no way to obtain human 5-lipoxygenase other than isolating it from a human organ. Thus, it is almost impossible to supply large amount of human 5-lipoxygenase continuously.

It has been tried to produce human 5-lipoxygenase by gene manipulation technique. The present inventors succeeded to isolate the cDNA of human 5-lipoxygenase from cDNA library prepared from human placenta and from human lung, and further succeeded to determine the entire base sequence of the gene encoding 5-lipoxygenase (Proceedings of the National Academy of Sciences, U.S.A., vol. 85, p. 26 (1988) and vol. 85, p. 3406 (1988)). Dixon et al isolated cDNA from HL-60 cells differentiated with dimethylsulfoxide and dexamethasone and determined the base sequence thereof (Proceedings of the National Academy of Sciences, U.S.A. vol. 85, p. 416 (1988)). However, it has not yet been succeeded to incorporate the gene in an microorganism and to make the microorganism produce human 5-lipoxygenase.

Accordingly, the object of the present invention is to provide a process of producing human 5-lipoxygenase in a large scale utilizing a microorganism.

The present inventors intensively studied to succeed in incorporating the gene encoding human 5-lipoxygenase in a cloning vector and to express the gene in a host cell to complete the present invention.

That is, the present invention provides a process of producing human 5-lipoxygenase comprising the steps of transforming E.coli with an expression vector containing a DNA region comprising a gene encoding a polypeptide with human 5-lipoxygenase activity; culturing the transformed E.coli at a temperature between 4 and 28°C; and recovering polypeptide with human 5-lipoxygenase activity from the E.coli.

By the present invention, a process of producing human 5-lipoxygenase with enzyme activity in a large scale utilizing a microorganism was provided.
Fig. 1 shows the base sequence of the DNA region encoding human 5-lipoxygenase, which was utilized in the example of the present invention, as well as the amino acid sequence encoded thereby;
Fig. 2 shows a process of constructing a DNA segment encoding human 5-lipoxygenase which further contains an *Eco* RI site immediately upstream the translation initiation codon ATG;
Fig. 3 shows the steps of constructing an expression vector pH5LOKC which expresses the human 5-lipoxygenase gene;
Fig. 4 shows the results of HPLC analysis of the protein produced by *E. coli* containing a vector pKC; and
Fig. 5 shows the results of HPLC analysis of the protein produced by *E. coli* containing the expression vector pH5LOKC.

The first step of the process of the present invention may be divided into three steps, that is, obtainment of human 5-lipoxygenase gene, construction of an expression vector containing the human 5-lipoxygenase gene and culturing of *E. coli* transformed with the expression vector. These steps will now be described in detail.

### (1) Obtainment of the Gene Encoding Human 5-Lipoxygenase

The DNA region encoding human 5-lipoxygenase, which is utilized in the process of the present invention, may be obtained from a natural source such as cDNA library obtained from human organs such as human placenta and lung. Some of these libraries are commercially available and the commercially available libraries may be employed as the source of human 5-lipoxygenase. It is also possible to use the DNA region encoding human 5-lipoxygenase which is synthesized by the method known in the art.

The desired human 5-lipoxygenase may be obtained from a cDNA library by the immunochemical method or by the probe hybridization method which *per se* are well-known in the art.

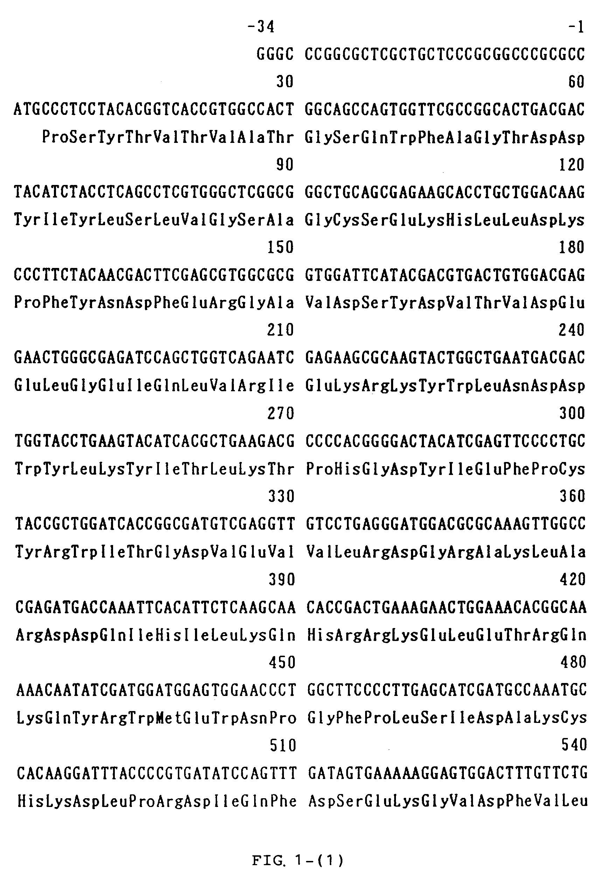

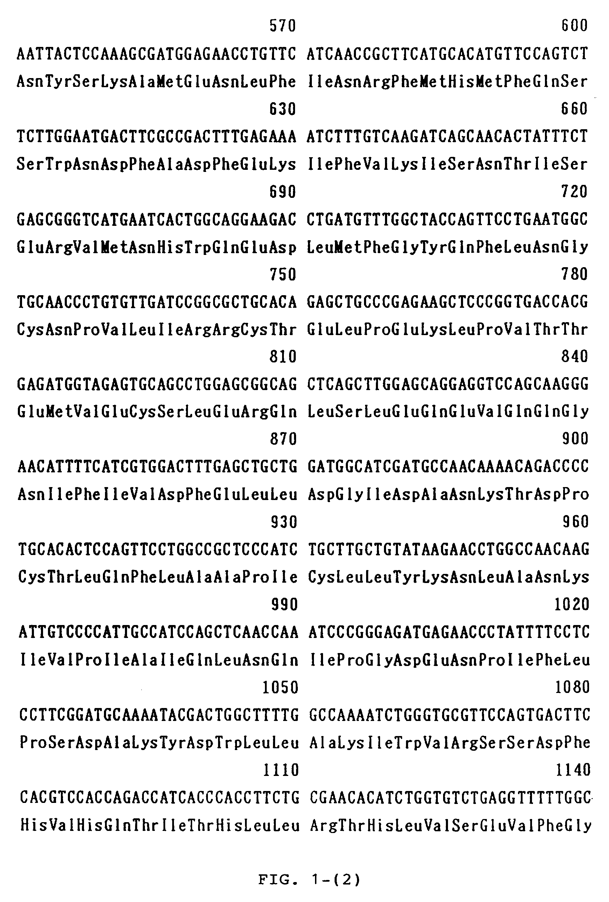

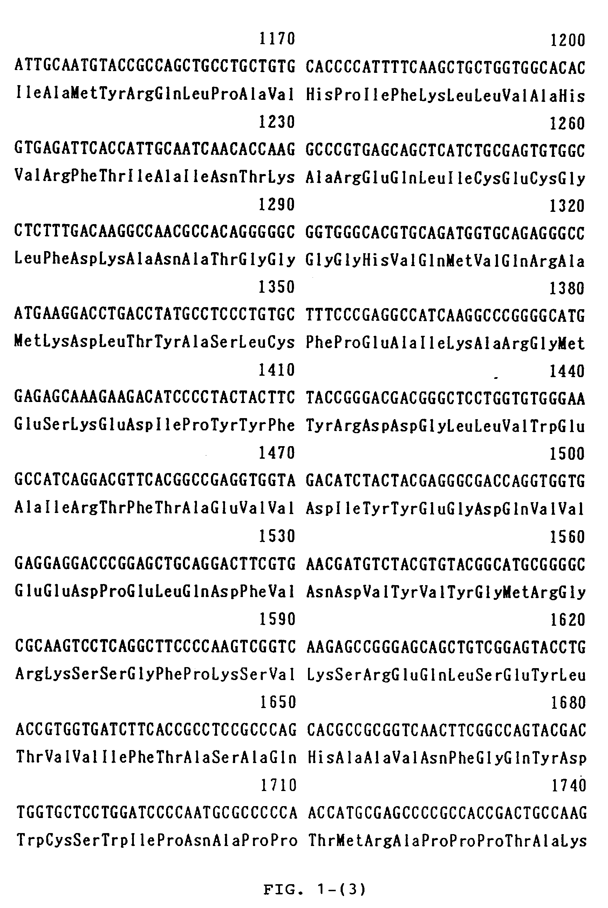

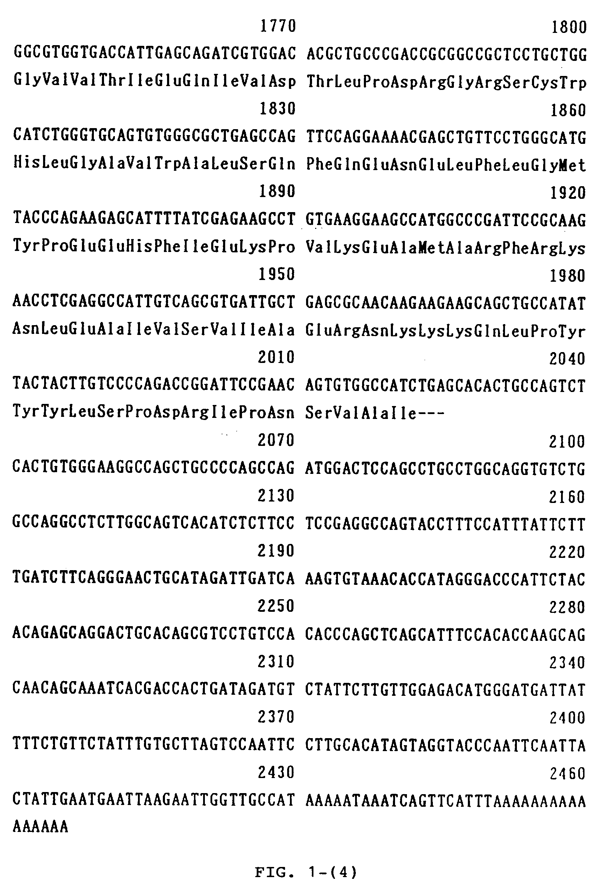

The entire base sequence of the DNA region encoding human 5-lipoxygenase, which was obtained in the Example later described as well as the amino acid sequence encoded by the DNA region are shown in Fig. 1. The region in the human 5-lipoxygenase gene, which is to be translated is from ATG of the base number 1 - 3 to TAG of the base number 2023 - 2025. However, since it has been proved that the N-terminal amino acid of the mature human 5-lipoxygenase is proline (Proceedings of the National Academy of Sciences, U.S.A. vol. 85, p.26 (1988)), the methionine upstream the N-terminal proline is considered to be cut off in the processing in the cell when a precursor is converted to the mature enzyme. The codon TAG of the base number 2023 - 2025 is a termination codon and it may be substituted with TAA or TGA.

It should be noted that in the production of an active protein by means of a genetic engineering technique, the protein is often produced in the form of a fused protein which contains amino acids originated from a linker necessary for constructing an expression vector. Thus, in the present invention, the production of such a fused protein is within the scope of the present invention as long as the fused protein has 5-lipoxygenase activity.

Further, it is well recognized by those skilled in the art that a physiological activity of a polypeptide may not be substantially influenced even if a small number of amino acids of the polypeptide are substituted with other amino acids, if a small number of amino acids are deleted from the polypeptide, or if a small number of amino acids are added to the polypeptide. Therefore, even if a small number of bases of the DNA region shown in Fig. 1 are substituted with other bases or deleted, or small number of bases are added to the base sequence so as to substitute a small number of amino acids of the polypeptide encoded by the region with other amino acids, to delete a small number of amino acids from the polypeptide or to add a small number of amino acids to the polypeptide, if the resulting polypeptide has an activity of human 5-lipoxygenase, the modified base sequence is construed to be a substantially the same base sequence as the base sequence shown in Fig. 1, and so is construed to be within the scope of the present invention. Further, in general, an amino acid is encoded by one or more codons. Therefore, another base sequence encoding the amino acid sequence shown in Fig. 1 is construed to be the same base sequence as that shown in Fig. 1.

In the examples later described, a gene encoding human 5-lipoxygenase was isolated from human placenta cDNA library (commercially available from Clontech) by the hybridization method using a synthetic probe in accordance with the description of Proceedings of the National Academy of Sciences, U.S.A., vol. 85, pp. 26 and 3406 (1988). The obtained cDNA (2.55 kbp) having a substantially complete length, had a repeated sequence with a size of 51 bp between *Xma* 1 sites, which does not occur in the natural human 5-lipoxygenase gene. Thus, the DNA fragment (422 bp) between the *Xma* 1 sites was cut out and was exchanged with a DNA fragment (371 bp) which did not contain the repeated base pairs and this DNA fragment was recombined in the correct orientation. To make it possible to control the number of bases between the translation initiation codon ATG required for the expression of the human 5-lipoxygenase gene and an Shine-Dalgarno sequence (hereinafter referred to as SD sequence) to which ribosome is attached, the non-translation region upstream the ATG codon was cut off and an *Eco* RI site was added immediately upstream the ATG codon.

### (2) Construction of Expression Recombinant

The expression vector used in the process of the present invention contains, in addition to the gene encoding human 5-lipoxygenase, replication origin for the replication in a host cell, a promoter such as *tac* promoter upstream the structural gene, a terminator such as *rrn*BT₁T₂ downstream the structural gene, an appropriate marker such as a gene giving drug resistance, and an SD sequence required for the *in vivo* translation as the conventional expression vector. Vectors containing these regions are commercially available and those commercially available vectors may be utilized as they are in the process of the present invention.

The expression vector used in the process of the present invention may be prepared by the following method. However, the method of preparation of the expression vector is not restricted to the following method.

The plasmid vector pkk223-3 (commercially available from Pharmacia) employed in the examples of the present invention hereinbelow described has ampicillin resistance (hereinafter referred to as Ap^{r}) as a selection marker, a strong *tac* promoter upstream the cloning site and a terminator downstream the cloning site. Since the pkk223-3 has a replication origin originated from pBR322, its copy number is not very large. Thus, in order to increase the copy number of pkk223-3, the replication origin thereof may be exchanged with the replication origin of, for example, vector plasmid pUC18 (commercially available from Takara Shuzo, Kyoto, Japan). This may be carried out as follows: First, pkk223-3 is digested with restriction enzymes *Pvu* I and *Pvu* II and the digest is subjected to the conventional agarose gel electrophoresis to separate the DNA fragments. The DNA fragments are then subjected to the DEAE paper method (Dretzen et al, Analytical Biochemistry, vol. 112, p. 295 (1981)) to collect the DNA fragments not containing the replication origin. pUC18 is also processed in the similar manner but the DNA fragments containing the replication origin are collected. The DNA fragments from both of the vectors are ligated by T₄DNA ligase to obtain a plasmid pKC. The human 5-lipoxygenase gene prepared in (1) is ligated to the thus prepared plasmid pKC to obtain an expression vector pH5LOKC which can express the human 5-lipoxygenase gene.

### (3) Production of Human 5-Lipoxygenase by Transformed E. coli

The transformation of *E. coli* with the thus prepared expression vector may be carried out by a method well-known in the art in accordance with, for example, the Method by Hanahan (Journal of Molecular Biology, vol. 166, p.557 (1983)). As the *E. coli*, K12 strains and their variants such as MV1184, JM109 and HB101 strains may be employed. The selection of the transformed *E. coli* may be carried out utilizing the selection marker.

In the process of the present invention, after the transformed *E. coli* is grown to a prescribed level, about 0.2 mM of isopropyl-β-D-thiogalactoside (hereinafter referred to as IPTG) is added to the culture medium to induce the expression of the human 5-lipoxygenase gene. Preferred medium used for the induction is LB medium containing 2% by weight of NaCl and 2% by volume of glycerol. The culture of the transformants may preferably be carried out at a temperature of 4 - 28°C, more preferably 10 - 20°C. If the culturing temperature is higher than 28°C, granules of the enzyme are formed in the host cell, so that the activity of human 5-lipoxygenase is lowered.

The human 5-lipoxygenase accumulated in the cell may be recovered and purified by the conventional method well-known in the art. For example, a cell suspension is centrifuged to collect the cells, and the collected cells are suspended again in a buffer solution containing a protease inhibitor, followed by ultrasonic disruption of the cells. The disrupted cells are centrifuged and the supernatant is then salted out with ammonium sulfate. Human 5-lipoxygenase is recovered in the protein fraction salted out at 30 - 60% saturated ammonium sulfate solution.

### [Examples]

The present invention will now be described in more detail by way of examples thereof. However, it should be understood that the examples are presented for the illustration purpose only and should not be interpreted in any restrictive way.

In the following example, unless otherwise specified, each step was carried out in accordance with T. Maniatis et al, Molecular Cloning, A Laboratory Manual (1982), Cold Spring Harbor. Further, the used restriction enzymes are commercially available and were used in accordance with the instructions by the manufacturer.

### (1) Construction of Recombinant Plasmid pH5LOU3 Encoding Human 5-Lipoxygenase

A DNA encoding human 5-lipoxygenase was isolated from human placenta cDNA library (commercially available from Clontech) by the hybridization method using a synthetic probe (Proceedings of the National Academy of Sciences, U.S.A., vol. 85, p. 26 and 3406 (1988)). The synthetic probe used here had a base sequence of base number 1849 - 2025 and the 220 bases downstream the termination codon, which are shown in Fig. 1. The base sequence of the thus isolated human 5-lipoxygenase as well as the amino acid sequence encoded thereby is shown in Fig. 1.

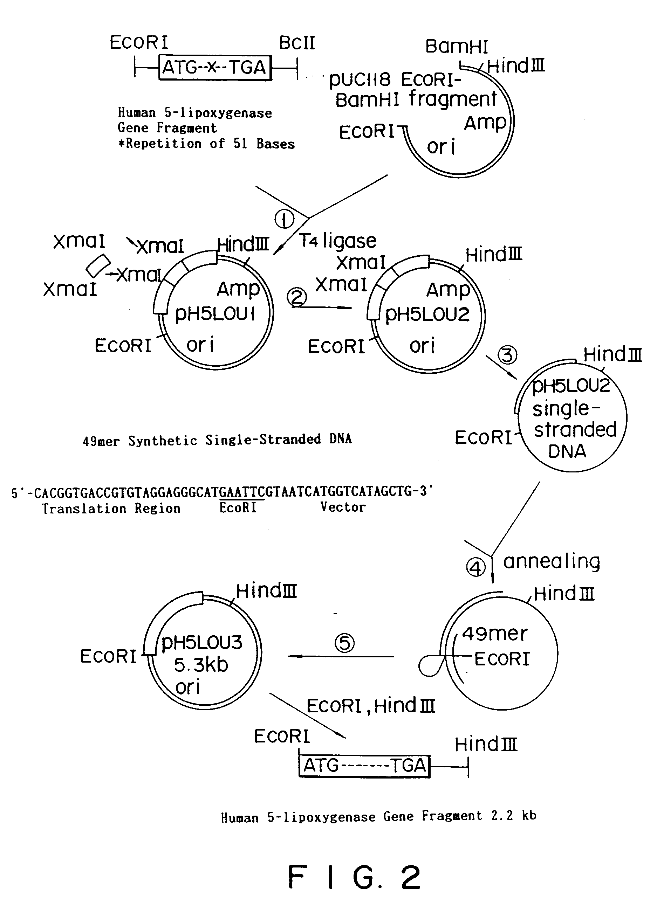

In the obtained cDNA with almost complete length (2.55 kbp), repeats of 51 base pairs from the base number 1229 to 1279 were contained between the base numbers 1228 and 1229. The repeated sequences were not contained in the other obtained cDNAS with incomplete length (2.2 kbp and 1.6 kbp). Therefore, it was concluded that the repeated sequences of 51 base pairs were artifacts, which were formed in the synthesis of cDNAS. To cut off the repeated sequences, the DNA fragment with a size of 2.55 kbp was dissolved in 40 µl of 25 mM Tris-HCl (pH 7.8) containing 50 mM NaCl and 10 mM MgCl₂, and 10 Units of *Bcl* I (commercially available from Pharmacia) was added to the solution, followed by incubation at 50°C for 3 hours to digest the cDNA fragment. The NaCl concentration of the resulting solution was adjusted to 100 mM and 10 units of *Eco* RI (commercially available from Nippon Gene) was added thereto, followed by incubation at 37°C for 3 hours to carry out the digestion reaction. The resulting reaction mixture was subjected to the phenol treatment and then to ethanol precipitation, and the obtained precipitate was dissolved in TE buffer (10 mM Tris-HCl containing 1 mM EDTA, pH 8.0). Then the plasmid vector pUC118 (commercially available from Takara Shuzo) was dissolved in 40 µl of a solution containing 100 mM NaCl and 10 mM MgCl₂ and 10 units each of *Eco*RI and *Bam* HI were added to the solution, followed by incubation at 37°C for 3 hours to carry out the digestion reaction. The resulting reaction mixture was subjected to phenol treatment and then to ethanol precipitation, and the precipitate was dissolved in TE buffer. To a mixture of 5 µl of the above-obtained solution containing the *Eco* RI - *Bcl* I DNA fragment (about 2.3 kbp) of the cDNA, 2 µl of the solution containing the *Eco* RI - *Bam* HI fragment of pUC118 (about 3.2 kbp), 1 µl of ligase buffer with 10-fold concentration and 1 µl of 10 mM ATP, 6 units of T₄ DNA ligase (commercially available from Takara Shuzo) was added and the resulting mixture was incubated at 14°C for 16 hours to carry out the ligation reaction. *E. coli* MV1184 strain was transformed with the resulting reaction mixture and a plasmid pH5LOU1 was obtained from an Ap^{r} colony (step 1 in Fig. 2). The DNA region of 422 bp interposed between two *Xma* I sites (containing 51 bp of the artifact) was cut out with *Xma* I (commercially available from IBI), and was exchanged with a DNA sequence with a length of 371 bp (not containing the artifact) interposed between *Xma* I sites in the cDNA with incomplete length. The DNA fragments were ligated with T₄ DNA ligase to obtain pH5LOU2 (step 2 in Fig. 2). The fact that pH5LOU2 contained the desired DNA fragment with about 2.2 kbp was confirmed by digesting pH5LOU2 with *Eco* RI and *Hind* III and then with *Pvu* II, followed by agarose gel electrophoresis. Thereafter, in order to adjust the number of bases between the translation initiation codon ATG necessary for the expression of the human 5-lipoxygenase gene and the SD sequence to 10, the non-translation region upstream the ATG was cut off and *Eco* RI site was added immediately upstream the ATG. To carry out this, a 49 mer single-stranded DNA having a base sequence below was synthesized by the conventional method with a DNA synthesizer 381A (commercially available from Applied Biosystems). 
5'-CACGGTGACCGTGTAGGAGGGCATGAATTCGTAATCATGGTCATAGCTG-3'
translation region    Eco RI    vector
In 20 µl of 50 mM Tris-HCl (pH 7.5) solution containing 10 mM MgCl₂, 10 mM dithiothreitol and 1 mM ATP, 32 pmol of the 49 mer single-stranded DNA was dissolved and 6 units of T₄ polynucleotidekinase (commercially available from Takara Shuzo) was added thereto, followed by the incubation at 37°C for 15 minutes to carry out the phosphorylation reaction. The reaction was stopped by heating the solution at 70°C for 10 minutes to inactivate the enzyme. Then *E. Coli* MV1184 strain was transformed with 1 µg of pH5LOU2 and Ap^{r} strain was collected to obtain a transformant. The thus obtained transformant was cultured in 3 ml of 2 x TY medium (bactotryptone 16g/l, yeast extract 10 g/l, NaCl 5 g/l, pH 7.6) containing 0.01% by weight of thiamine and 150 µg/ml of ampicillin at 37°C. About 1 hour later, a helper phage M13K07 was infected at a multiple infection ratio of 10, and after additional 1 hour, kanamycin was added to a level of 70 µg/ml. The resultant was cultured under shaking for additional 18 hours at 37°C. One milliliter of this culture liquid was transferred to an Eppendorf's tube and was centrifuged at 12,000 rpm for 5 minutes. To the supernatant, was added 200 µl of 2.5 M aqueous NaCl solution containing 20% by weight of polyethyleneglycol 6000 and the resulting mixture was left to stand for 15 minutes at room temperature, followed by centrifugation at 12,000 rpm for 2 minutes. The obtained precipitate was completely dissolved in 100 µl of TE buffer and the resulting solution was subjected to phenol treatment and then ethanol precipitation, followed by drying under reduced pressure to obtain a single stranded DNA (step 3 in Fig. 2). The preparation of site-specific variant utilizing the oligonucleotide hereinbelow described was carried out in accordance with the description in Oligonucleotide-directed *in vitro* Mutagenesis System Handbook published by Amersham. Eight picomoles of the above-described 49 mer synthetic oligonucleotide was annealed with the DNA region of 10 µg of the single-stranded DNA of human 5-lipoxygenase gene, which region contained 5′-end translation region and *Eco* RI linker (step 4 in Fig. 2), and a double-stranded DNA was prepared using DNA polymerase I Klenow fragment and T₄DNA ligase under the presence of dATP, dCTPα-S, dGTP and dTTP. The DNA chains which did not contain thionucleotide were digested with restriction enzyme *Nci* I and the digest was treated with exonuclease III. Then the double-stranded DNA pH5LOU3 which did not contain the base pairs in interest was prepared using DNA polymerase I and T₄DNA ligase under the presence of dATP, dCTP, dGTP and dTTP (step 5 in Fig. 2) to obtain a variant which did not have 5′ non-translation region of human 5-lipoxygenase and in which *Eco* RI site was added immediately upstream the translation initiation codon ATG of human 5-lipoxygenase. The DNA sequence encoding the N-terminal region of the human 5-lipoxygenase in pH5LOU3 is as follows, which was confirmed by the dideoxy sequence method using a 17 mer synthetic oligonucleotide with a sequence of 5′-ATGTAGTCGTCAGTGCC-3′ as a primer: 
5'-CAGCTATGACCATGATTACGAATTCATGCCCTCCTACACGGTCACCGTG-3'
GTCGATACTGGTACTAATGCTTAAGTACGGGAGGATGTGCCAGTGGCAC
vector          Eco RI MetProSerTyrThrValThrVal
As a result, it was confirmed that pH5LOU3 contained DNA encoding human 5-Lipoxygenase.

### (2) Construction of Recombinant Plasmid pH5LOKC Expressing Human 5-lipoxygenase Gene

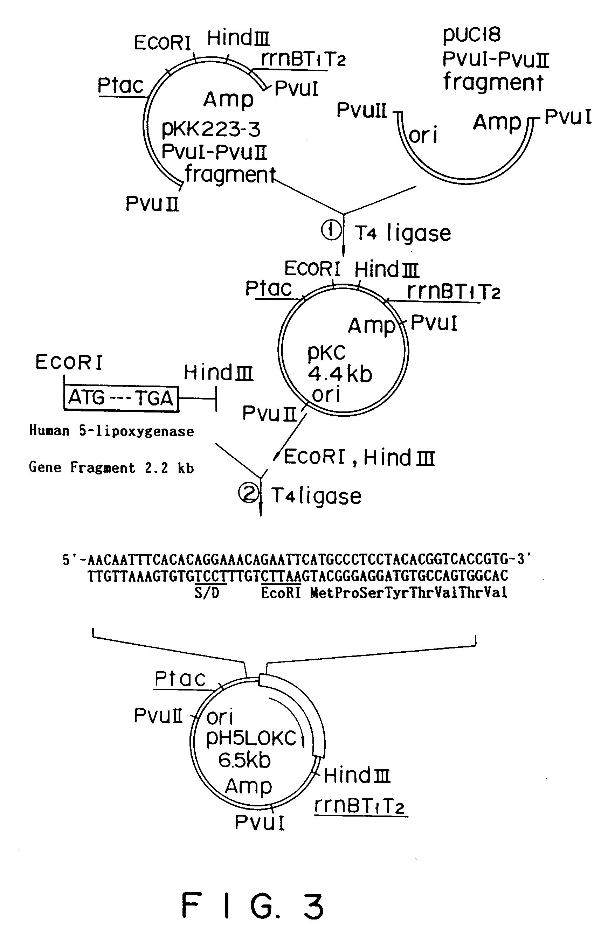

The replication origin of plasmid pKK223-3 (commercially available from Pharmacia Japan) containing *tac* promoter was exchanged with the replication origin of pUC18 (commercially available from Takara Shuzo) with which a large number of copies of the plasmid may be obtained, and an expression vector plasmid pKC was constructed. That is, 10 µg of pKK223-3 was digested with 10 unites each of restriction enzymes *Pvu* I and *Pvu* II and the resultant was separated by agarose gel electrophoresis and a DNA fragment with a size of about 2.9 kbp was collected by the DEAE paper method. Similarly, 10 µg of pUC18 was digested with 10 units each of *Pvu* I and *Pvu* II and a DNA fraction of about 1.4 kbp containing the replication origin was isolated. The DNA fragments prepared from pKK223-3 and from pUC18 were ligated with T₄DNA ligase to obtain a plasmid pKC (step 1 in Fig. 3). The pH5LOU3 containing the DNA region encoding human 5-lipoxygenase prepared in Example (1) was digested with *Eco* RI and *Hin*d III and the DNA encoding human 5-lipoxygenase was isolated. pKC was digested with *Eco* RI and *Hin*d III and the digest was ligated with *Eco* RI - *Hin*d III DNA fragment (about 2.2 kbp) using T₄DNA ligase to obtain pH5LOKC (step 2 in Fig. 3). *E. coli* MV1184 strain was transformed with the thus prepared pH5LOKC and a transformant was obtain from an Ap^{r} colony. The transformant was cultured in LB medium at 37°C for 18 hours to amplify pH5LOKC. It was confirmed that the thus obtained plasmid DNA contained the desired DNA of about 2.2 kbp by the digestion with *Eco* RI and *Hin*d III and subsequent agarose gel electrophoresis. It was confirmed by the dideoxy sequence method that the DNA region in p5HLOKC, which region encodes the N-terminal region of human 5-lipoxygenase, had the following sequence: 
5'-AACAATTTCACACAGGAAACAGAATTCATGCCCTCCTACACGGTCACCGTG-3'
TTGTTAAAGTGTGTCCTTTGTCTTAAGTACGGAGGATGTGCCAGTGGCAC
S/D EcoRI MetProSerTyrThrValThrVal
The *E. coli* JTS872-L1 containing the plasmid pH5LOKC was deposited in Fermentation Research Institute of Japan under the Budapest Treaty under the accession number of FERM-BP 2750.

### (3) Production of Human 5-Lipoxygenase by E. coli containing pH5LOKC

*E. coli* MV1184 strain was transformed with the recombinant plasmid pH5LOKC obtained in Example (2). An Ap^{r} colony was inoculated to 10 ml of LB medium (pH 7.7) and was cultured at 28°C for 18 hours. Ten milliliters of this culture was added to 1 liter of LB medium containing 2% by weight of NaCl and 2% by volume of glycerol and the culture was continued at 20°C for another 10 hours under shaking. When the absorbance at 550 nm of the culture reached to about 0.8, IPTG was added to a level of 0.2 mM and the culture was continued another 15 hours at 20°C under shaking. When the absorbance at 550 nm of the culture liquid reached to about 6, the culture liquid was centrifuged to obtain about 10 g of the cells. The thus obtained cells were suspended in 50 ml of 50 mM phosphate buffer (pH 7.0) containing 0.1 M NaCl, 2 mM EDTA, 3 mg of soybean trypsin inhibitor, 1 mM phenylmethylsulfonyl fluoride and 1 mM dithiothreitol and 20 mg of lysozyme was added thereto. The resulting suspension was incubated at 37°C for 15 minutes to decompose the cell walls, followed by destroying the cells by ultrasonic disruption. The suspension containing the disrupted cells were centrifuged at 12,000 x g at 4°C for 10 minutes and the supernatant was subjected to salting out with ammonium sulfate. The protein fractions salted out at 30 - 60% saturated ammonium sulfate solution were dialyzed against 50 mM Tris-HCl (pH 8.0) containing 20% by volume of glycerol, 2 mM EDTA and 2 mM dithiothreitol. After the protein fractions were suspended in Laemmli's sample buffer, the suspension was subjected to SDS-PAGE and the gel was stained with Coomassie Briliant Blue. As a result, a polypeptide band at a position of about 80 kd which corresponded to human 5-lipoxygenase was detected. This band was not detected when *E. coli* MV1184 strain containing the plasmid pKC not containing the human 5-lipoxygenase gene was used. Thus, it was proved that the *E. coli* having pH5LOKC was producing human 5-lipoxygenase in a large amount.

### (4) Measurement of Human 5-Lipoxygenase Activity

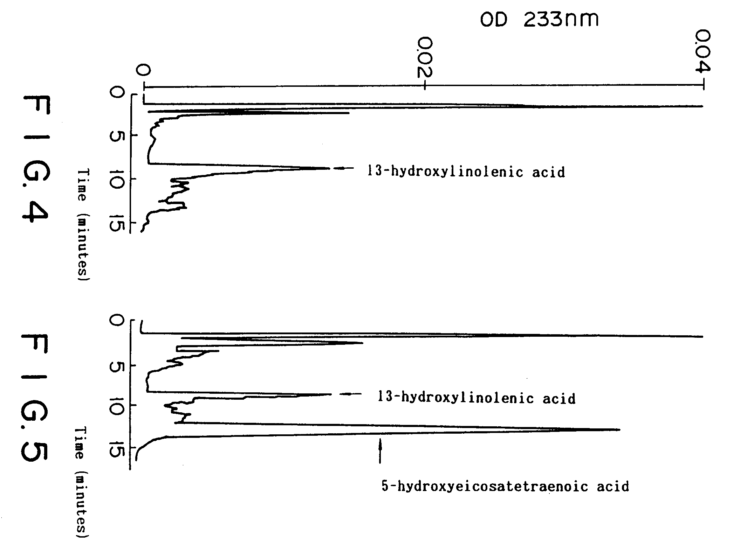

To 5 µl of yolk phosphatidyl choline (100 mg/ml, hexane, commercially available from Sigma), were added 350 µl of 0.1 M Tris-HCl (pH 8.0) containing 1 mM EDTA and 5 ml of diethylether, and the resulting mixture was subjected to ultrasonication under nitrogen flow while evaporating diethylether to obtain a uniform phosphatidyl choline suspension. On the other hand, arachidonic acid was reacted with hydrogen peroxide under the presence of cupric chloride to obtain hydroperoxyeicosatetraenoic acid (Proceedings of the National Academy of Sciences, U.S.A. vol. 82, p. 6040 (1985)). In an Eppendorf's tube, 20 µl of 0.5 M Tris-HCl (pH 8.0) containing 10 mM CaCl₂ and 10 mM ATP was placed, and then 10 µl of the phosphatidyl choline suspension and 70 µl of the human 5-lipoxygenase fraction obtained in Example (3) (230 µg in terms of protein) were added thereto, followed by preincubation at 30°C for 5 minutes. To this mixture, 2 µl of 4 mM arachidonic acid containing 10 mM synthetic hydroperoxyeicosatetraenoic acid was added to initiate the reaction and the resulting mixture was incubated at 30°C for 10 minutes. Then 300 µl of methanol containing 2 µg/ml of 13-hydroxylinolenic acid as an internal standard substance, which was preliminarily cooled to -20°C was added to the mixture. One microliter of 1 N acetic acid was then added to the mixture and the resulting mixture was vigorously stirred, followed by centrifugation at 8000 x g for 10 minutes. From the supernatant with a volume of 400 µl, 100 µl was taken and was analyzed by HPLC to determine the level of 5-hydroxyeicosatetraenoic acid. The conditions of HPLC were as follows:
Column: CAPCELLPAK C18 SG120 4.6 mm x 150 mm (commercially available from Shiseido, Tokyo, Japan)
Solvent: methanol/water/acetic acid = 75/25/0.01 (v/v/v)
Detection: absorbance at 233 nm
Flow Rate: 1.0 ml/min.
Temperature: 38°C
The results of the measurement are shown in Figs. 4 and 5. As shown in Fig. 5, the protein produced by the *E. coli* containing pH5LOKC, that is, the *E. coli* in which human 5-lipoxygenase gene is incorporated exhibited 5-lipoxygenase activity. In contrast, as shown in Fig. 4, the protein produced by the *E. coli* containing pKC, that is, the *E. coli* containing only the vector plasmid did not exhibit 5-lipoxygenase activity.

### (5) Structure of Human 5-Lipoxygenase Produced by E. coli

The protein fractions containing human 5-lipoxygenase obtained in Example (3) were purified by the method in accordance with Rouzer et al., Proceedings of the National Academy of Sciences, U.S.A., vol. 82, p. 6040 (1985). The amino acid sequence of the N-terminal region of the thus purified protein was determined using a liquid phase protein sequencer 477A commercially available from Applied Biosystems. As a result, the amino acid sequence of the N-terminal region of the human 5-lipoxygenase produced by *E. coli* completely agreed with that of the human 5-lipoxygenase isolated from human leucocytes.

## Claims

1. A process for producing human 5-lipoxygenase which comprises the steps of
transforming E.coli with an expression vector containing a DNA region comprising a gene encoding a polypeptide with human 5-lipoxygenase activity;
culturing the transformed E.coli at a temperature between 4 to 28°C; and
recovering produced polypeptide with human 5-lipoxygenase activity from the E.coli.

2. A process according to claim 1 wherein the DNA region encoding the polypeptide comprises an EcoRI site immediately upstream of a translation initiation codon.

3. A process according to claim 1 or claim 2 wherein the expression vector comprises one or more of:
(i) a tac promoter upstream of the gene encoding the polypeptide;
(ii) a rrn BT₁T₂ terminator downstream of the gene encoding the polypeptide;
(iii) a marker gene;
(iv) a Shine-Dalgarno sequence.

4. A process according to any one of claims 1 to 3 wherein the expression vector is pH5LOKC as shown in Fig. 3

5. A process according to any one of claims 1 to 4 wherein E.coli is one of
(i) a K12 strain;
(ii) a MV1184 strain;
(iii) a JM109 strain; or
(iv) an HB101 strain.

6. A process according to any one of the preceding claims wherein the culturing is at a temperature between 10 and 20°C.

7. A process according to any one of the preceding claims wherein human 5-lipoxygenase is recovered collecting E.coli cells;
disrupting the E.coli cells;
separating the insoluble cell debris from material in solution;
salting out proteins with ammonium sulphate; and
recovering human 5-lipoxygenase from the protein fraction salted out in a 30-60% saturation ammonium sulphate solution.

## Patentansprüche

1. Verfahren zur Herstellung menschlicher 5-Lipoxygenase, umfassend die folgenden Schritte:
Transformieren von E.coli mit einem Expressionsvektor, der einen DNA-Bereich enthält, der ein für ein Polypeptid mit menschlicher 5-Lipoxygenase-Aktivität kodierendes Gen umfaßt;
Kultivieren des transformierten E.coli bei einer Temperatur zwischen 4 und 28°C; und
Gewinnen des produzierten Polypeptids mit menschlicher 5-Lipoxygenase-Aktivität aus E.coli.

2. Verfahren nach Anspruch 1, worin der für das Polypeptid kodierende DNA-Bereich eine EcoRI-Stelle unmittelbar stromauf von einem Translationsinitiations-Kodon umfaßt.

3. Verfahren nach Anspruch 1 oder 2, worin der Expressionsvektor einen oder mehrere der folgenden umfaßt:
(i) einen tac-Promotor stromauf von dem für das Polypeptid kodierenden Gen;
(ii) einen rrn-BT₁T₂-Terminator stromab von dem für das Polypeptid kodierenden Gen;
(iii) ein Markergen;
(iv) eine Shine-Dalgarno-Sequenz.

4. Verfahren nach irgendeinem der Ansprüche 1 - 3, worin der Expressionsvektor pH5LOKC ist, wie in Fig. 3 dargestellt.

5. Verfahren nach irgendeinem der Ansprüche 1 - 4, worin E.coli einer der folgenden Stämme ist:
(i) ein K12-Stamm;
(ii) ein MV1184-Stamm;
(iii) ein JM109-Stamm; oder
(iv) ein HB101-Stamm.

6. Verfahren nach irgendeinem der vorangegangenen Ansprüche, worin das Kultivieren bei einer Temperatur zwischen 10 und 20°C erfolgt.

7. Verfahren nach irgendeinem der vorangegangenen Ansprüche, worin menschliche 5-Lipoxygenase gewonnen wird durch:
Sammeln von E.coli-Zellen;
Aufbrechen der E.coli-Zellen;
Abtrennen der unlöslichen Zellbruchstücke vom Material in Lösung;
Aussalzen von Proteinen mit Ammoniumsulfat; und
Gewinnen menschlicher 5-Lipoxygenase aus jener Proteinfraktion, die aus einer zu 30 - 60% gesättigten Ammoniumsulfat-Lösung ausgesalzt wurde.

## Revendications

1. Procédé de production de la 5-lipoxygénase humaine qui comprend les étapes de
transformation de E. coli avec un vecteur d'expression contenant une région d'ADN comprenant un gène codant pour un polypeptide avec une activité de 5-lipoxygénase humaine ;
culture de E. coli transformé à une température entre 4 et 28°C ; et
récupération du polypeptide produit avec l'activité de la 5-lipoxygénase humaine de E. coli.

2. Procédé selon la revendication 1 dans lequel la région d'ADN codant pour le polypeptide comprend un site Eco RI immédiatement en amont d'un codon d'initiation de la traduction.

3. Procédé selon la revendication 1 ou la revendication 2 dans lequel le vecteur d'expression comprend un ou plusieurs parmi :
(i) un promoteur tac en amont du gène codant pour le polypeptide ;
(ii) un terminateur rrn BT₁T₂ en aval du gène codant pour le polypeptide ;
(iii) un gène marqueur ;
(iv) une séquence de Shine-Dalgarno.

4. Procédé selon l'une quelconque des revendications 1 à 3 dans lequel le vecteur d'expression est pH5LOKC comme montré à la Figure 3.

5. Procédé selon l'une quelconque des revendications 1 à 4 dans lequel E. coli est l'un parmi
(i) une souche K12 ;
(ii) une souche MV1184 ;
(iii) une souche JM109 ; ou
(iv) une souche HB101.

6. Procédé selon l'une quelconque des revendications précédentes dans lequel la culture est à une température entre 10 et 20°C.

7. Procédé selon l'une quelconque des revendications précédentes dans lequel la 5-lipoxygénase humaine est récupérée en collectant les cellules de E. coli ;
en détruisant les cellules de E. coli ;
en séparant les débris de cellules insolubles du matériau en solution ;
en relarguant les protéines avec du sulfate d'ammonium ; et
en récupérant la 5-lipoxygénase humaine de la fraction de protéine relarguée dans une solution saturée à 30 à 60% de sulfate d'ammonium.
